# EUROPEAN PATENT APPLICATION

(11) **EP 4 115 735 A1**
(43) Date of publication of application: **11.01.2023**
(21) Application number: 20922531.7
(22) Date of filing: 04.03.2020
(51) Int. Cl.: A01N 43/16, A01N 25/00, C08B 37/06, A01P 3/00

(54) **METHOD FOR PREPARING A FORMULATION FOR INDUCING AN IMMUNE RESPONSE OF A PLANT TO A PATHOGEN, AND FORMULATION**

(71) Applicant: Gênica Inovação Biotechnológica S.A., 13414-157 Piracicaba (BR)
(72) Inventor: MARTINS FONSECA REIS, Fernando, 78731-621 Rondonópolis, São Paulo (BR); RECCHIA, Gustavo Henrique, 13147-780 Piracicaba, São Paulo (BR)
(74) Representative: Crugnola, Pietro
(86) International application number: PCT/BR2020/050068
(87) International publication number: WO 2021/174322

(57) **Abstract**

This invention refers to a method to prepare a formulation having as active ingredients straight chain oligogalacturonide compounds of galacturonic [1→4]-α-D-acid residues with Polymerization Degree between 3-8 units. This formulation is intended for the foliar treatment of a plant by inducing the acquired systemic resistance (ASR), decreasing its susceptibility to pathogen *Phakopsora pachyrhiz,* which causes the disease, commonly known as Asian rust.

## Description

### TECHNICAL FIELD

. This invention refers to a method to prepare a formulation having as active ingredients straight chain oligogalacturonide compounds of galacturonic [1→4]-α-D-acid residues with Polymerization Degree between 3-8 units. This formulation is intended for the foliar treatment of a plant by inducing the acquired systemic resistance (ASR), decreasing its susceptibility to pathogen *Phakopsora pachyrhiz,* which causes the disease, commonly known as Asian Rust.

### INVENTION BACKGROUND

. Currently, the Asian rust control is conducted by using the following methods: follow the fallowing (period of at least 60days without live soybean plants in the field); use cultivars with resistance gene(s); start sowing in the beginning of the recommended time; use early cultivars (escape); and apply chemical fungicides.

. Such chemical control has been made through site-specific fungicides (which act on a single point of the fungus metabolism), and multi-site (acting in more than one point of the fungus metabolism). Once such fungicides are specific in their toxicity, they can be absorbed by the plant and tend to present systemic properties (McGrath, 2004; Technical Circular Letter 144 by EMBRAPA).

. The current fungicides belong to the following groups: demethylation inhibitors (DMI, "triazoles" - tebuconazole, cyproconazole, prothioconazole, difenoconazole, epoxiconazole and tetraconazole), quinine outside inhibitors (QOI, "strobilurins" - azoxystrobin, trifloxystrobin, picoxystrobin, metominostrobin and pyraclostrobin), succinate dehydrogenase inhibitors (SDHI, "carboxamides" - fluxapyroxad, bixafene, benzovindiflupir, fluindapir and impirfluxam), dithiocarbamate (mancozeb), chloronitrile (chlorothalonil) and inorganic (copper oxychloride) (Godoy eta/., 2017a; Technical Circular 138 by EMBRAPA). The number of registered fungicides for the control of Asian rust have increased from five in 2002 to 117 in 2015 (Godoy *et al*., 2016).

. Since the first years of use for disease control, the fungicides have intensively differentiated their efficacy, being that resistant populations of *Phakopsora pachyrhiz* less sensitive to such molecules are frequently identified (FRAC, 2017; Klosowski et *al*., 2015; Schmitz et *al*., 2013; Simões et *al*., 2017). In addition, the strong dependence of such tool is a continuous source of concern with respect to the risks to the applicator's and the environment health. Among the criteria adopted for application, the following is recommended: the use of more than one group of fungicides per cycle, with different modes of action; to prioritize the applications during the flourishing, pod formation and grain filling stages, with view to the periods of increased susceptibility; under severe epidemic conditions, perform from three to five applications at 10-day intervals (Sinclair and Hartman, 1995); and conduct preventive applications still in the vegetation period, in the pre-closure of the sowing lines, considering the climate and foreseeing the infection (Deuner *et al*., 2009).

. On the other hand, the oligogalacturonides (hereinafter also referred to by their acronym OGs) have shown the capacity to elicit several defense responses in the plant, including the induction of transcription of genes that are essential for this process (Ferrari et al. 2003). In the plant, the degradation of the pectin which composes the cell wall by invading microorganisms results in the release of such oligomers of galacturonic [1→4]-α-D-acid residues, the accumulation of which will trigger such defense processes. However, the OGs have also shown to affect several aspects of the plants growth and development (Darvill *et al*., 1992), such as the elongation of pea stem segments (Branca eta/., 1988), the flourishing (Marfà eta/., 1991) and the organogenesis of roots in tobacco explants (Bellicampi et *al*., 1993), a process associated with the OGs antagonism with phytohormone auxin. In addition, the OGs are also considered as endogenous signs when released at low quantity as a consequence of the cell wall remodeling during the development of the vegetable cells (Ferrari *et al*., 2003). Consequently, the form on which the plants can discriminate between low physiological doses of OGs and high quantities produced during the interaction with the pathogens was not yet duly clarified. Thus, it is crucial to follow the dosage and application positioning recommendations so that important aspects of the plant development and preparation are not negatively affected.

. In *Arabidopsis* seedling, the responses triggered by the OGs overlap those activated by MAMPs (microbial associated molecular patterns). The transcriptomes of seedlings treated with OGs and flg22 (peptide related to the active epitope of flagellin), indicate a wide overlap of responses in the first moments following treatment (30-60 min.). Such effectors activate a series of responses which are independent from the ethylene-, salicylic acid- (SA) and jasmonate- (JA) mediated signaling pathways and induce the phosphorylation of two MAP Kinase proteins, AtMPK3 and AtMPK6, the second one being necessary for the early expression of defense genes and to induce resistance against pathogen *Botrytis cinerea.* In addition, the OGs and flg22 produce an oxidative response mediated by enzyme (NADPH)- oxidase AtRbohD, which is partially responsible for the subsequent deposition of callose.

. The *in vivo* preparation of OGs by *Arabidosis* plants was explored by expressing a chimeric protein derived from the fusion of a polygalacturonase obtained from fungus *Fusarium phyllophillum* (FpPG) and from a PGIP of *Phaseolus vulgaris* (PvPGIP2). By expressing the chimeric construction under the control of promoters regulated by β-estradiol and by preserved gene R PR-1, active OGs were accumulated, with the Polymerization Degree ranging from 6-13, extending the resistant to the attach of fungus *Botrytis cinerea* and bacteria *Pseudomonas syringae* and *Pectobacterium carotovorum.*

. In face of the above mentioned considerations, it was verified the need to synthesize OGs *in vitro* in large scale with view to the formulation of a resistance inducing product for the foliar treatment of a culture of agricultural interest, such as, for example, soybean.

. There isn't in the state-of-the-art, any OGs intended for the induction of a systemic immune response in the soybean culture, to decrease the susceptibility of the culture to attach by *Phakopsora pachyrhiz,* the fungus which causes Asian rust. In addition, there are no reports of commercial products with OGs as active ingredient.

### SUMMARY DESCRIPTION OF THE INVENTION

. Thus, the object of this invention is to provide a method to prepare a formulation containing galacturonic [1→4]-α-D-acid oligomers, with Polymerization Degree between 3-8, for the foliar treatment of plants in the phenological development stages where they are more susceptible to the pathogen attack. The formulation elicits a wide range of defense responses which include the accumulation of phytoalexins, glucanases and chitinases, callose deposition, of oxygen and nitric oxide reactive species.

. This invention does not refer to an alternative to the conventional chemical treatments currently available in the market. It provides an additional tool for the disease control, once it allows the desired control levels to be reached with a lower number of chemical fungicide applications per culture cycle.

. This invention refers to a method to prepare a formulation intended to induce an immune response by a plant to a pathogen, comprising the steps of:
(a) purifying oligogalacturonide compounds, from the product of the partial enzymatic hydrolysis of polygalacturonic acid during the pectin refining process,
   on which the hydrolysis reaction is conducted by a pectinase complex (pectin methylesterases, endopolygalacturonases and exopolygalacturonases), obtained from *Aspergillus niger,* at a temperature ranging from 26°C to 36°C, stirring ranging from 100 to 200 rpm and pH between 3.8 and 4.5;
(b) boiling the obtained solution at a temperature of 100°C for 10 min. for enzymatic inactivation;
(c) diluting the obtained solution at 0.5% with 50 mM NaOAc solution;
(d) adding EtOH to the obtained solution, obtaining a solution with a concentration of 11%;
(e) maintaining the solution of step (d) incubated for 16 hours at a temperature of 4°C so as to precipitate the oligogalacturonides;
(f) centrifuging the solution of step (e) at 30,000 g for 30 min., obtaining a formulation containing powdered oligogalacturonides and soluble in water;
(g) re-suspending the compound in water so as to obtain a solution with a concentration of 1 mg × mL⁻¹ × ha⁻¹; and
(h) applying the compound in a plant susceptible to a pathogen.

. During step (a) of the method of this invention the enzymatic concentration is 100 U, the temperature is 30°C, stirring is at 150 rpm and pH is 4.00 to 4.05.

. In step (g) of the method of this invention, it is applied on the plant eliciting a defense response by the plant by inducing the transcription of genes that are essential for this process.

. The biotrophic pathogen to be treated by the method of this invention is *Phakopsora pachyrhiz* and the plant to be treated is preferably soybean.

. Through the method of this invention a formulation is obtained comprising, as the active ingredient, the compound of molecular structure 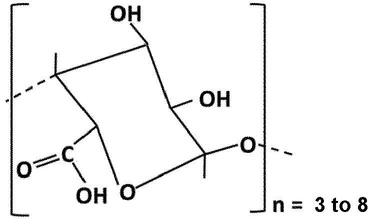 which refers to straight chain oligogalacturonide compounds of galacturonic [1→4]-α-D-acid residues with Polymerization Degree between 3-8 (Chemical formula: (C₆H₈O₆)ₙ).

. The concerned formulation further comprises:
- from 70 to 80% of inactive polygalacturonic acid;
- from 10 to 15% of active fraction of oligomers with Polymerization Degree between 3-8 units;
- from 10 to 12% of NaOAc;
- from 0.1 to 0.5% of inactivated enzyme composed by pectinase complex of pectin methylesterases, endo- and exo- polygalacturonases, isolated from *Aspergillus niger;* and
- from 0.01 to 0.05% of inactivated BSA carrier protein.

. The formulation preferably comprises:
- 74.71% of inactive polygalacturonic acid;
- 14.01% of active fraction of oligomers with Polymerization Degree between 3-8 units;
- 10.99% of NaOAc;
- 0,26% of inactivated enzyme composed by pectinase complex of pectin methylesterases, endo- and exo- polygalacturonases, isolated from *Aspergillus niger;* and
- 0,03% of inactivated BSA carrier protein.

. Most of the defense responses and effects on the vegetable development exercised by the OGs require a specific Polymerization Degree. Thus, it is evident that in order to activate specific response standards, OGs with also specific Polymerization Degree are required, being that for this proposal it is established that OGs with a Polymerization Degree between 3-8 are required to activate the immune response in soybean able to reduce the progression of the infection by fungus *Phakopsora pachyrhiz,* responsible for causing the Asian soybean rust disease. It is important to emphasize that the Polymerization Degree between 3-8 presents an inducing action of resistance in face of other diseases caused by the infection through microorganisms, especially those, the parasitism of which has a biotrophic character, not only with focus on the soybean culture but, also, other cultures of agricultural interest.

. Finally, this invention is focused on the development of a single product, intended to induce resistance, however, the formulation obtained herein can be incorporated as an additive for the formulation of other commercial products such as fertilizers, growth inducers or even fungicides.

### BRIEF DESCRIPTION OF THE FIGURES

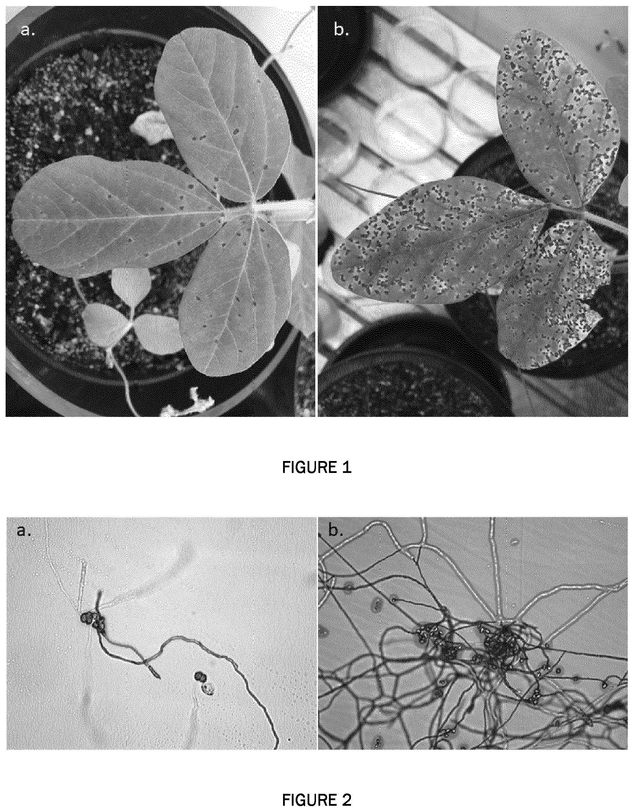

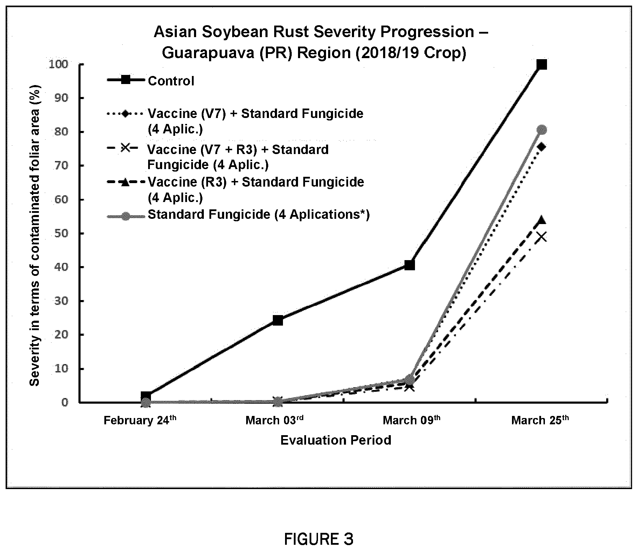

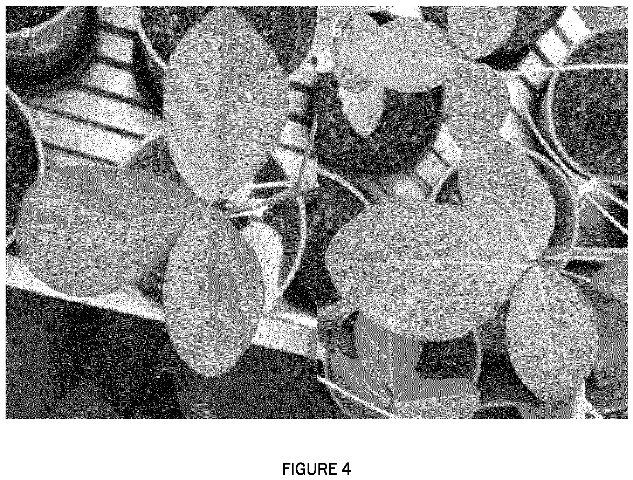

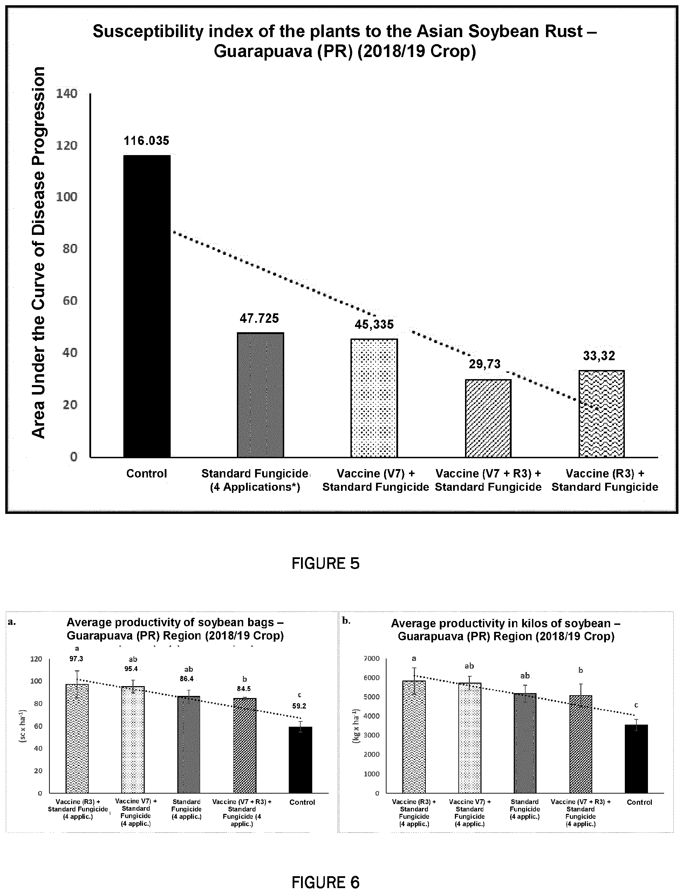

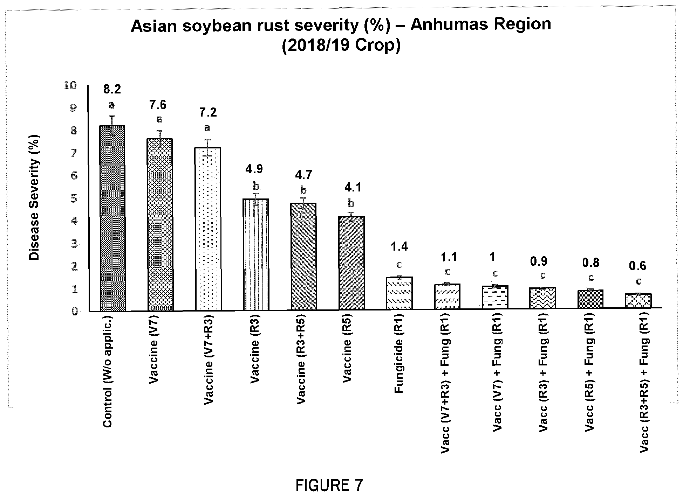

. Figure 1 - Photos of Conquista-MG cultivar plants 30 days after the application of the oligomer formulation obtained by this invention and inoculation as a suspension containing 5 × 10⁵ spores/ml of *Phakopsora pachyrhiz.*
. Figure 2 - Photos of *Phakopsora pachyrhiz* spores germination test in 1.5% agar-water medium collected 25 days after inoculation.
. Figure 3 - Chart showing the Asian soybean rust severity progression curve in terms of percentage (%) of contaminated foliar area in plants of cultivar K-5616.
. Figure 4 - Photos of cultivar Monsoy IPRO M8372 IPRO plants after 19 days of treatment with the oligomer formulation obtained by this invention.
. Figure 5 - Chart showing the calculation of the area under the curve of disease progression (AACPD) for the index which indicates the disease susceptibility level after the treatments in plants of cultivar K-5616.
. Figure 6 - Chart showing the average productivity data of cultivar K-5616 obtained in the study area in Guarapuava (PR) for the 2018/19 Crop.
. Figure 7 - Chart showing the Asian soybean rust severity degree in terms of percentage (%) of contaminated foliar area in plants of cultivar Agroeste 3730 Intacta.

### DETAILED DESCRIPTION OF THE INVENTION

. As previously mentioned, there was a need to develop an innovative, profitable and safe solution for the control and handling of the Asian soybean rust. Thus, this invention discusses the methods to obtain an oligomer formulation which activate the defense mechanisms of the plant against the imminent attack of the pathogen, creating an additional tool to handle with the disease which allows to decrease the number of fungicide applications. In this case, the focus is no longer the chemical control of the inoculum conducted by the traditional fungicides (with high toxicity potential and which pose risk to the human, animal and environment health), but the plant. Thus, the risks inherent to the selection of resistant populations of *Phakopsora pachyrhiz* are decreased due to the continuous exposure to the same groups of molecules which constitute the formulation base of such fungicides.

. Thus, this invention was developed with view to a method to manufacture an oligomer formulation with straight chain compounds of galacturonic [1→4]-α-D-acid residues with Polymerization Degree between 3-8 units.

. Such oligomer formulation obtained by the concerned method is preferably a water-soluble solid product (Dry powder), intended for foliar application in soybean culture, inducing resistance in the plant in face of the attack by pathogen *Phakopsora pachyrhiz.*

. It is important to emphasize that although the oligomer formulation shows potential to activate systemic immune responses, this type of response tends to have a transient character, requiring a continuous exposure the effector molecule so that it lasts for a longer period. However, oligomers have another crucial role in the vegetable physiology. Being a direct product of pectin degradation, essential compound of the cell wall, apoplastic variations in the concentration of such molecules may activate the transduction of cytoplasmic signs which directly affect the expression of genes related to the cellular development, with potential impact on the plant development and preparation. Thus, it is crucial to pay attention to the final product concentration in solution, and that the applications are conducted only in the periods of higher susceptibility of the culture to the pathogen in order to avoid an overload to the plant - transition from vegetative to reproductive (V7/8), flourishing (R1), pod formation (R3) and grain filling (R5).

. As the treatment focus is the plant, not the fungus, it is essential that the chemical control with fungicides, although at lower scale, is still maintained so as to prevent an increase of the inoculums pressure in the field. Such strategy provides an additional tool in the fight against the disease, once it allows the desired control levels to be reached with a lower number of chemical fungicide applications per culture cycle, decreasing the negative impact caused by phytotoxicity, risk to human health and environmental contamination.

### Conducted tests

. All the tests were conducted with soybean plants maintained in controlled growth chambers (24±1°C, photoperiod 14/10 hours, light intensity of 280 µmol × m⁻² × s⁻¹), the foliar applications of the compound (1mg . ml⁻¹) being conducted with a manual sprayer, in stage V4 of phenological development of the plant, around 12 hours before inoculation with solution of 5 × 10⁵ spores/ml of *Phakopsora pachyrhiz.*

. The plants were followed-up on a daily basis throughout the experimental period, being the visual scoring scale proposed by Godoy et al. (2006), employed in order to verify the disease severity progression (%). It is worth emphasizing that for new proofs of concept, the plants were maintained up to the flourishing stage (R1) once the growth chamber size was not fit for plants beyond this development stage.

. The control plants are those not treated with the oligomer formulation obtained by this invention or fungicide.

### Test 1

. Plants from cultivar Conquista-MG, very susceptible to the disease, were submitted. 11 days after inoculation, only the pustules present in the non-treated plants presented sporulation; plants treated with the oligomer formulation of this invention produced on average 26.3x105 spores/ml, while for the non-treated control plants, the count was 9,4x106 spores/ml of *Phakopsora pachyrhiz,* i.e., 3.58 times more spores.

. The viability of such spores was also estimated. The obtained spores were inoculated in agar-water medium and evaluated after 25 days. For plants treated with the oligomer formulation of this invention, only 49.6% of the spores sprouted (Figure 2a), while for the non-treated control plants such rate was higher, 73% (Figure 2b).

. 30 days after the treatment, the plants from cultivar Conquista-MG treated with the oligomer formulation of this invention presented a maximum of 7% of the foliar area with lesions (Figure 1a), while the non-treated control plants presented up to 78% of the area with lesions (Figure 1b).

### Test 2

. Plants from cultivar IPRO M8372, with intermediate potential of susceptibility to the disease, were submitted. At the end of the experiment (19 days), all the inoculated plants exhibited some disease symptoms. However, the disease severity showed to be different in both treatments. For plants treated with the oligomer formulation of this invention, 80% of the contaminated leaves presented between 0.2 - 2% of their total areas with lesions (Figure 4a), among the non-treated leaves, in their turn, 80% presented from 2-18% of their areas with lesions (Figure 4b). The maximum contamination in treated leaves was 7%.

### Test 3

. Conducted in the region of Guarapuava (PR). Three compound application treatments were considered therein: (i) an application at pre-flourishing stage (V7/8), (ii) an application in the beginning of the pod forming stage (R3), and (iii) two applications of the oligomer formulation of the present invention in both development stages (V7/8 + R3), always concomitantly with chemical fungicides in compliance with the standard application calendar of the experimental area in the farm (Table 1).

**Table 1. Fungicide application routine in agronomic efficiency test conducted in the region of the municipality of Guarapuava (PR) in the 2018/19 Crop.**

| Fungicide Applications | | |
|---|---|---|
| Product | Date | Dose/ha |
| Fox and Cuprodil | 01/16/2019 | 0.4 and 1.0 |
| Elatus and Sumilex and Bendazol | 01/29/2019 | 0.2 and 0.5 and 1.0 |
| Cronnos and Bendazol and Cuprodil | 02/11/2019 | 2.25 and 1.0 and 1.0 |
| Unizeb Gold and Nativo | 02/26/2019 | 1.25 and 0.5 |

. The adopted cultivar was K-5616. The experimental design system was parcels subdivided with randomization in factorial system. 24 parcels were defined, divided into four blocks, subdivided according to the phenological development stage of the plant where the application of the oligomer formulation obtained by this invention would be conducted. The sizes of the parcels (blocks) were 5 × 3 (m), containing 7 rows, being the two more external rows and 0.50 m of each side used as border to isolate the parcels. The treatments were fully randomized. An average population density of 300 thousand plants × ha⁻¹ was used. The final application dose of the oligomer formulation obtained by this invention was 1 mg/ml, corresponding to 16.8% in 100 L/ha.

. Due to the strong presence of the disease in the region, it was possible to collect disease severity data (%) over the months of February and March 2019. Through the Chart in Figure 3, it is possible to verify the Asian soybean rust severity progression curve in terms of percentage (%) of contaminated foliar area in plants of cultivar K-5616.

. The weekly evaluation was conducted according to the visual scoring scale proposed by Godoy *et al*. (2006), where five treatments were conducted: (i) control with no form of disease control; (ii) one application of the oligomer formulation obtained by this invention, in stage V7 of the plant development with four additional fungicide applications; (iii) two applications of the oligomer formulation obtained by this invention, in stages V7 and R3 of plant development with four additional fungicide applications; (iv) one application of the oligomer formulation obtained by this invention, in stage R3 of plant development with four additional fungicide applications; and (v) four fungicide applications.

. For the other treatments, parcels which received two applications of the oligomer formulation of this invention (V7/8 + R3) concomitantly with the standard application of fungicides presented the lowest disease severity levels (49.1%). Single applications showed to be more efficient when conducted during the reproduction stage, when the plant is under higher inoculum pressure (54.3% for application in R3), being that the application only in V7/8 exhibited similar control levels (75.6%) in parcels where only the standard application of fungicides was conducted (80.7%).

. Figure 5 illustrates the result a chart with the calculation of the area under the curve of disease progression (AACPD) for the index which indicates the disease susceptibility level after the treatments in plants of cultivar K-5616 grown in the region of the municipality of Guarapuava (PR) during the 2018/19 Crop.

. Figure 6 is a chart showing the average productivity data of cultivar K-5616 obtained in the study area in Guarapuava (PR) for the 2018/19 Crop. Figure 6a represents the average Productivity in soybean bags per hectare of planted area (sc x ha¹); Figure 6b represents the average productivity in kilos of soybean per hectare of planted area (kg × ha⁻¹).

### Test 4

. Conducted in the region of Piracicaba (SP), five compound application treatments were considered: (i) one application in the pre-flourishing stage (V7/8); (ii) one application in the beginning of the pod forming stage (R3), (iii) one application in the grain filling stage (R5); and (iv) and (v) two applications, in V7/8 + R3, and in R3 + R5, respectively.

. In addition, parcels where only applications of the oligomer formulation of this invention were conducted, only one fungicide application (Fox^{®}, Bayer) in R1, and parcels with both treatments, were adopted.

. The cultivar adopted was Agroeste 3730 Intacta. The experimental design system was parcels subdivided with randomization in factorial system. 96 parcels were defined, divided into four blocks, subdivided according to the phenological development stage of the plant where the application of the oligomer formulation obtained by this invention and/or fungicide would be conducted. The sizes of the parcels (blocks) were 5 × 3 (m), containing 7 rows, being the two more external rows and 0.50 m of each side used as border to isolate the parcels. The treatments were fully randomized. An average population density of 300 thousand plants × ha⁻¹ was used. The final application dose of the oligomer formulation obtained by this invention was 1 mg/ml, corresponding to 16.8% in 100 L/ha.

. Due to the strong drought in the region during the first two weeks of December/2018, and over the entire month of January/2019, the plants suffered a strong impact on their development. With view to minimize the phytotoxicity effects as a result of the high temperatures, only one fungicide application was conducted in 13/Feb (0.4 L/ha). As a result of the rain intensification only at the end of the month of March/2019, the rust was only registered in the end of the crop development cycle (03/16/2019), being that only one disease severity evaluation (%) was possible.

. Through the chart of Figure 7, it is possible to verify the Asian soybean rust severity degree in terms of percentage (%) of contaminated foliar area in plants of cultivar Agroeste 3730 Intacta.

### Conclusion

. This data evidence that the soybean treatment with biologically active fractions of the oligomer formulation of this invention affects the colonization by *Phakopsora pachyrhiz,* with direct impact on the disease progression severity (%) in the field.

. Therefore, it can be concluded that this invention is innovative and ecologically sustainable, once it makes use of a chemical effector which naturally occurs in plants for the treatment of a disease for which the handling is conducted almost exclusively based on chemical fungicides with high toxicity degree and which pose risks to the human, animal and environment health. The addition of this tool to the integrated handling techniques of the disease, particularly as a complement to the usual chemical control practices, provides the possibility to decrease the number of fungicide applications throughout the crop development cycle.

## Claims

1. METHOD TO PREPARE A FORMULATION TO INDUCE AN IMMUNE RESPONSE of a plant to a pathogen, **characterized by** the fact that it comprises:
(a) purifying oligogalacturonide compounds, from the product of the partial enzymatic hydrolysis of polygalacturonic acid during the pectin refining process,
on which the hydrolysis reaction is conducted by a pectinase complex (pectin methylesterases, endopolygalacturonases and exopolygalacturonases), obtained from *Aspergillus niger,* at a temperature ranging from 26°C to 36°C, stirring ranging from 100 to 200 rpm and pH between 3.8 and 4.5;
(b) boiling the obtained solution at a temperature of 100°C for 10 min. for enzymatic inactivation;
(c) diluting the obtained solution at 0.5% with 50 mM NaOAc solution;
(d) adding EtOH to the obtained solution, obtaining a solution with a concentration of 11%;
(e) maintaining the solution of step (d) incubated for 16 hours at a temperature of 4°C so as to precipitate the oligogalacturonides;
(f) centrifuging the solution of step (e) at 30,000 g for 30 min., obtaining a formulation containing powdered oligogalacturonides and soluble in water;
(g) re-suspending the compound in water so as to obtain a solution with a concentration of 1 mg × mL⁻¹ × ha⁻¹; and
(h) applying the compound on a plant susceptible to a pathogen.

2. METHOD according to claim 1, **characterized by** the fact that in step (a) the enzymatic concentration is 100 U, the temperature is 30 °C, stirring is at 150 rpm and pH is of 4.00 to 4.05.

3. METHOD according to claim 1, **characterized by** the fact that the solution of step (g) is applied on the plant eliciting a defense response in the plant by inducing the transcription of genes essential for such process.

4. METHOD according to claim 1, **characterized by** the fact that the biotrophic pathogen is *Phakopsora pachyrhizi.*

5. FORMULATION, **characterized by** the fact that it is obtained by the method as described by claims from 1 to 4, its active ingredient comprises oligomers of galacturonic [1→4]-α-D-acid residues, containing Polymerization Degree between 3-8 units.

6. FORMULATION, according to claim 5, **characterized by** the fact that it comprises:
- from 70 to 80% of inactive polygalacturonic acid;
- from 10 to 15% of active fraction of oligomers with Polymerization Degree between 3-8 units;
- from 10 to 12% of NaOAc ;
- from 0.1 to 0.5% of inactivated enzyme composed by pectinase complex of pectin methylesterases, endo- and exo-polygalacturonases, isolated from *Aspergillus niger;* and
- from 0.01 to 0.05 % of inactivated BSA carrier protein.

7. FORMULATION, according to claim 6, **characterized by** the fact that it comprises:
- 74.71% of inactive polygalacturonic acid;
- 14.01% of active fraction of oligomers with Polymerization Degree between 3-8 units;
- 10.99% of NaOAc;
- 0,26% of inactivated enzyme composed by pectinase complex of pectin methylesterases, endo- and exo-polygalacturonases, isolated from *Aspergillus niger;* and
- 0,03% of inactivated BSA carrier protein.

## Amended claims

### Amended claims under Art. 19.1 PCT

1. METHOD TO PREPARE A FORMULATION TO INDUCE AN IMMUNE RESPONSE of a plant susceptible to a pathogen, **characterized by** the fact that it comprises:
(a) purifying oligogalacturonide compounds, from the product of the partial enzymatic hydrolysis of polygalacturonic acid during the pectin refining process,
on which the hydrolysis reaction is conducted by a pectinase complex (pectin methylesterases, endopolygalacturonases and exopolygalacturonases), obtained from *Aspergillus niger,* at a temperature ranging from 26°C to 36°C, stirring ranging from 100 to 200 rpm and pH between 3.8 and 4.5;
(b) boiling the obtained solution at a temperature of 100°C for 10 min. for enzymatic inactivation;
(c) diluting the obtained solution at 0.5% with 50 mM sodium acetate solution;
(d) adding ethanol to the obtained solution, obtaining a solution with a concentration of 11%;
(e) maintaining the solution of step (d) incubated for 16 hours at a temperature of 4°C so as to precipitate the oligogalacturonides;
(f) centrifuging the solution of step (e) at 30,000 g for 30 min., obtaining a formulation containing powdered oligogalacturonides and soluble in water;
(g) re-suspending the compound in water so as to obtain a solution with a concentration of 1 mg.mL⁻¹.ha⁻¹;

2. METHOD according to claim 1, **characterized by** the fact that in step (a) the enzymatic concentration is 100 U, the temperature is 30 °C, stirring is at 150 rpm and pH is of 4.00 to 4.05.

3. FORMULATION obtained by the method as described by claims from 1 to 2, **characterized by** the fact that its active ingredient comprises oligomers of galacturonic [1→4]-α-D-acid residues, containing Polymerization Degree between 3-8 units.

4. FORMULATION, according to claim 3, **characterized by** the fact that it comprises:
- from 70% to 80% in weight of inactive polygalacturonic acid;
- from 10% to 15% in weight of active fraction of oligomers with Polymerization Degree between 3-8 units;
- from 10% to 12% in weight of sodium acetate;
- from 0.1% to 0.5% in weight of inactivated enzyme composed by pectinase complex of pectin methylesterases, endo- and exo-polygalacturonases, isolated from *Aspergillus niger;* and
- from 0.01% to 0.05% in weight of inactivated BSA carrier protein.

5. FORMULATION, according to claim 4, **characterized by** the fact that it comprises:
- 74.71% in weight of inactive polygalacturonic acid;
- 14.01% in weight of active fraction of oligomers with Polymerization Degree between 3-8 units;
- 10.99% in weight of sodium acetate;
- 0,26% in weight of inactivated enzyme composed by pectinase complex of pectin methylesterases, endo- and exo-polygalacturonases, isolated from *Aspergillus niger;* and
- 0,03% in weight of inactivated BSA carrier protein.

6. USE OF A FORMULATION as defined by claims 3 to 5, **characterized by** the fact that it is applied on a plant eliciting a defense response to a possible pathogen by inducing the transcription of essential genes.

7. USE according to claim 6, **characterized by** the fact that the biotrophic pathogen is *Phakopsora pachyrhizi.*
